Europäisches Patentamt

European Patent Office

Office européen des brevets

(11) Publication number: **0 502 042 B1**

## EUROPEAN PATENT SPECIFICATION

(45) Date of publication of patent specification: **12.04.95**  (51) Int. Cl.⁶: **A61K 9/48,** A61K 9/66, A61K 47/10, A61K 31/55

(21) Application number: **90917309.8**

(22) Date of filing: **23.11.90**

(86) International application number: **PCT/GB90/01821**

(87) International publication number: **WO 91/07950 (13.06.91 91/13)**

### (54) PHARMACEUTICAL COMPOSITIONS.

(30) Priority: **24.11.89 GB 8926612**

(43) Date of publication of application: **09.09.92 Bulletin 92/37**

(45) Publication of the grant of the patent: **12.04.95 Bulletin 95/15**

(84) Designated Contracting States: **BE CH DE DK IT LI SE**

(56) References cited:
**EP-A- 0 120 248
EP-A- 0 152 292
DE-A- 2 614 864
GB-A- 2 185 887**

**Manufacturing Chemist, vol. 60, n 12, December 1989 (Woolwich, London, GB) A.P. Launchbury et al "The development of temazepam gelthix", page 38-40**

(73) Proprietor: **PHARMACIA S.p.A.
Via Robert Koch, 1.2
I-20152 Milano (IT)**

(72) Inventor: **LAUNCHBURY, Alfred Paul
51A Station Road
New Barnet, Herts. EN5 1PR (GB)**
Inventor: **MORTON, Frank Samuel Short
8260 141st Street North
Seminole, FL 34646 (US)**
Inventor: **ROWE, Dennis
19 Branscombe Drive
Wootton Bassett
Swindon
Wilts. SN5 8HR (GB)**

(74) Representative: **Woods, Geoffrey Corlett et al
J.A. KEMP & CO.
14 South Square
Gray's Inn
London WC1R 5LX (GB)**

EP 0 502 042 B1

**Description**

The present invention relates to improvements in and relating to pharmaceutical compositions in unit dosage form.

Temazepam is a benzodiazepine with a combination of properties which have secured its widespread clinical acceptance as a hypnotic and oral premedicant. It has sleep inducing and anxiolytic properties, low toxicity (the oral LD 50 in mice is almost 1000mg/kg), no active metabolites, a short half life and no significant drug interactions. Both the efficacy of the drug as a hypnotic/premedicant, and prompt clearance of the drug to give early recovery and avoid "hangover" characteristics, depend upon rapid and complete absorption.

Both hypnotic and premedicant activity are dependant on the rate of rise of drug blood levels, a rapid steep rise being essential. Conventional formulations of temazepam in tablets or hard gelatine capsules, which depend on dissolution of temazepam powder in the gut, are therefore not suitable. For this reason, a softgel capsule comprising temazepam dissolved in a liquid hydrophilic fill was developed. This formulation, containing temazepam dissolved in the water miscible solvent polyethylene glycol 400 (PEG 400), permitted the drug to be completely absorbed within 40 to 60 minutes, resulting in prompt sleep induction or premedicant anxiolysis. The early completion of the absorption phase also permitted a subsequent steep fall in blood levels (half life about 1.3 hours) during the distribution phase, which was responsible for the early smooth recovery seen with this drug. It also resulted in an elimination half life of 8 to 10 hours.

This combination of desirable properties led to wide clinical acceptance of the liquid filled temazepam softgel capsule. However, there have recently been reports that the liquid fill of the capsules lends itself to use by intravenous drug abusers. It also became apparent that it was the convenience of the liquid fill, and not an inherent attractiveness of temazepam itself, that lay behind the growing abuse.

Against this background a way of formulating temazepam was sought which would meet the criteria of removing the convenience afforded to drug abusers by the original fill liquid, whilst retaining the good bioavailability and pharmacokinetic properties of the drug. A further criterion was that the eventual solution adopted should be "non-punitive". The existence of potential sclerosants in the capsule fill was therefore to be avoided, because of the hazard this would present to the persistent intravenous drug abuser who, despite all possible deterrents, nevertheless managed to inject the new formulation.

A variety of options presented themselves, but were rejected:

(a) Suspension of the temazepam in a variety of thick lipophilic carriers reduced the potential for abuse, but failed to give the required rapid dissolution profile.

(b) Colloidal silicon dioxide was identified as a potential gelling agent for the original fill liquid, but in order to obtain a capsule fill of the required viscosity some 12 to 15% silicon dioxide was required. The strength of the gel was found to be highly dependant on the quantity of water present, which made the manufacture of reproducible gels difficult. The release of temazepam from such fills was in any event found to be poor.

(c) Polyvinylpyrrolidone was identified as a further potential gelling agent. However, it was found to be difficult to achieve reproducible gelling with this material.

(d) The possibility of using a neutralised carbomer was examined. This had the potential for allowing a gel to be produced which would be difficult to syringe at neutral pH, but would still allow rapid drug release at gastric pH. However, manufacturing problems were encountered with this option owing to time dependant viscosity increases. Also, the dissolution of temazepam from these mixes was inferior to dissolution from the original liquid-fill product owing to the slow reversal of the gel at gastric pH.

EP-A-0120248 discloses soft gelatin capsules having a gelatin shell containing 4 to 40%, based on dry shell weight, of sorbitol or sorbitan as softener, and a capsule filling which comprises an active substance suspended in polyethyleneglycol, of which at least 50% by weight is a polyethylene glycol of average molecular weight 600, and up to 20% by weight of glycerol and/or propylene glycol. EP-A-0152292 describes gelatin capsules containing a fill material which comprises acetaminophen suspended in a vehicle comprising a first polyethylene glycol of average molecular weight 400 to 1000 and a second polyethylene glycol of average molecular weight from 1000 to 10000, a surfactant and a viscosity modifier. DE-A-2614864 describes soft gelatin capsules filled with digoxin in a solution comprising lactose, liquid and solid polyethylene glycols with an average molecular weight of 300 to 6000, glycerine and/or propylene glycol.

There is now provided, in accordance with the present invention, a pharmaceutical unit dosage form comprising a soft gelatin capsule shell or a two-piece hard gelatin capsule and a fill comprising a benzodiazepine dissolved or suspended in a gel, wherein the gel has a dropping point (°C), prior to encapsulation, of at least 46 and comprises at least 63% by weight of polyethylene glycol 600, at least 2% by weight of polyethylene glycol 4000 or 6000 and at least 21% by weight of an intermediate polyethylene

2

EP 0 502 042 B1

glycol, the gel consisting of polyethylene glycol.

It is highly surprising in several respects that the present invention meets the various criteria set out earlier. Firstly, previous attempts to thicken the original liquid capsule fill of PEG 400 with higher molecular weight polyethylene glycols had resulted in formulations which were still capable of being expelled from a syringe at ambient temperature. It was therefore not to be expected that a composition comprising a major proportion of a low molecular weight polyethylene glycol as base could be rendered non-injectable.

Secondly, it was not to be predicted that polyethylene glycol 6000 would be suitable as a thickener because in general, when dissolved in a lower molecular weight polyethylene glycol, PEG 6000 tends to crystallise out. The effect of this is to diminish the viscosity of the solution. It was found, however, that the combination of an intermediate polyethylene glycol with the higher molecular weight polyethylene glycol and the base polyethylene glycol reduces the tendency of the higher PEG to crystallise whilst not adversely affecting viscosity or drug dissolution.

The polyethylene glycols forming the gel are polymers of the general formula $H(OCH_2CH_2)_nOH$ and are commonly referred to as macrogols, or PEGs. The number following each polyethylene glycol corresponds to its average molecular weight. Thus polyethylene glycol 600 (or PEG 600) has an average molecular weight range of from 570 to 630; polyethylene glycol 4000 (PEG 4000) has an average molecular weight range of from 3700 to 4500; and polyethylene glycol 6000 (PEG 6000) has an average molecular weight range of from 5600 to 7000.

By "intermediate" polyethylene glycol is meant a PEG having an average molecular weight between 600 and 4000. One example is polyethylene glycol 1500 (PEG 1500) which has a molecular weight range of from 1300 to 1600.

The amount by weight of PEG 600 in the gel is at least 63% by weight, for example from 63 to 75% or from 65 to 75% by weight. Other suitable ranges include from 65 to 73%, from 67 to 70% and from 67 to 68%. An amount of from 67 to 68% by weight of PEG 600 is particularly preferred.

The amount of PEG 4000, when present in the gel, is at least 2% by weight, for example from 2 to 12%, or from 4 to 10% by weight. An amount of from 7 to 10% by weight of PEG 4000 is particularly suitable.

The amount of PEG 6000, when present in the gel, is at least 2% by weight, for example from 2 to 7% or from 4.3 to 6.9% by weight. An amount of from 4 to 6% by weight of PEG 6000 is particularly suitable.

At least 2% by weight of PEG 6000 in the gel is generally preferable to at least 2% by weight of PEG 4000.

The intermediate polyethylene glycol is present in the gel in an amount of at least 21% by weight, for example from 21 to 33% or from 25 to 30% by weight. Other suitable ranges include from 21 to 30%, from 22 to 29% and from 25 to 28%. An amount of from 27 to 28% by weight of PEG 1500 is particularly preferred.

Examples of two preferred gel formulations are:

(i) PEG 600, 68.0%; PEG 1500, 28.0%; PEG 6000, 4.0%; and

(ii) PEG 600, 65%; PEG 1500, 25%; PEG 4000, 10%.

The gel consisting of the polyethylene glycol blend has a drop point of at least 46°C before it is encapsulated in the unit dosage form of the invention. Owing to the nature of the encapsulation process, the drop point is no greater than 75°C. Typically is has a maximum value of 60°C, more preferably 50.3°C. Typically the drop point is at least 48°C, for example at least 48.2°C. Preferably it lies in the range of 48°C to 60°C, for example 48.2°C to 50.3°C. The drop point is the temperature at which the gel, when suspended in solid form and heated, begins to drop. It may be measured using a standard Metler FP83 dropping point apparatus using a sample cup conforming to DIN 51801. Heating rate 1°C per minute.

The fill may further comprise conventional ingredients such as glycerol, other co-solvents such as propylene glycol, or colorants, e.g. pigments or dyes. When glycerol is included, it is typically present in an amount of from 2 to 20%, for example from 3 to 15%, preferably from 4 to 12%, more preferably from 4.5 to 8% by weight based on the fill prior to encapsulation. As will be discussed, the glycerol content of the fill tends to rise after encapsulation owing to migration of a small amount of glycerol from the shell to the hydrophilic fill.

In a preferred embodiment of the present invention the fill includes chlorophyll, since the green coloration that this imparts to the capsule contents provides a visual deterrent to would-be drug abusers. The amount of chlorophyll used should be sufficient to make the capsule contents deep green. In a 10mg temazepam capsule containing approximately 300mg of fill, this typically means an amount of from 0.2 to 0.3mg chlorophyll.

The capsule shell comprises gelatin. It is either a conventional two-piece hard gelatin capsule or a soft gelatin capsule shell. The soft gelatin capsule shell further comprises a plasticiser for the gelatin, such as

3

glycerin, propylene glycol or sorbitol. The plasticiser is suitably present in an amount of from 10 to 40% by weight, for example from 12 to 35% by weight, more preferably from 15 to 25% by weight.

The soft gelatin capsule shell typically further incorporates an embrittlement inhibiting composition comprising sorbitol in admixture with one or more sorbitans and, possibly, other polyhydric alcohols. The amount of sorbitol/sorbitan(s) in the shell is suitably from 4 to 25% by weight, for example from 6 to 20% by weight, preferably from 9 to 15% by weight. When other polyhydric alcohols are present the total amount of sorbitol and sorbitan in the embrittlement inhibiting composition is suitably from 45 to 75% by weight, for example from 55 to 66% by weight. The other polyhydric alcohols are typically hydrogenated saccharides.

Suitable materials for introducing the embrittlement inhibiting composition into the soft gelatin capsule shell are concentrated aqueous solutions of polyhydric alcohols derived from the hydrolysis and partial hydrogenation of glucose syrup. Examples thereof include the commercially available material sold under the Trade name "Anidrisorb 85/70". This product has the following typical analysis.

| Components (% by weight of concentrated aqueous solution) | | | | |
|---|---|---|---|---|
| D-Sorbitol | Sorbitans | Other polyols | Mannitol | Water |
| 24-45 | 20-30 | 20-25 | 0-6 | 13-20 |

The capsule shell, whether hard or soft, suitably also comprises other conventional ingredients such as colouring agents (pigments or dyestuffs), anti-oxidants or preservatives. It is particularly preferred in the present invention to include chlorophyll in the shell to maintain product identity with the original liquid filled capsules. The chlorophyll is suitably present in the soft gelatin shell in an amount of from 0.05 to 0.5% by weight, in particular from 0.08 to 0.3% by weight, more preferably about 0.1% by weight. A suspension of $TiO_2$ in glycerol is also suitably included in the soft gelatin shell, the ratio of $TiO_2$ to glycerol typically being of the order of 1:3. Such a suspension suitably forms from 0.5 to 2% by weight of the soft gelatin capsule shell, more preferably about 1% by weight.

Any member of the benzodiazepine group of compounds may be dissolved or suspended in the polyethylene glycol gel and encapsulated in the hard or soft gelatin capsule shell. Temazepam is a preferred benzodiazepine for use in the present invention.

The invention further provides a process for preparing a pharmaceutical unit dosage form as hereinbefore defined which process comprises

(a) preparing a polyethylene glycol blend by combining at least 63% by weight of polyethylene glycol 600, at least 2% by weight of polyethylene glycol 4000 or 6000 and at least 21% by weight of an intermediate polyethylene glycol,

(b) forming a fill by dissolving or suspending a benzodiazepine in the polyethylene glycol blend, and

(c) encapsulating the fill in a soft gelatin capsule shell or a two-piece gelatin capsule.

The blend of polyethylene glycols for the gel of the capsule filling is prepared by a standard method, typically involving combining the three components of different molecular weight in a jacketed mixing vessel maintained at a temperature equal to, or above, the melting point of the highest molecular weight polyethylene glycol. For example, the polyethylene glycol 600 is placed in a jacketed mixing vessel, to which the intermediate polyethylene glycol, such as polyethylene glycol 1500, and either polyethylene glycol 4000 or polyethylene glycol 6000 are added. The mix is heated to about 75°C, with stirring. Stirring is then continued at this temperature until a clear solution is obtained.

Glycerol may be included with the polyethylene glycol blend in the capsule fill. It is suitably introduced into the fill by being combined with the polyethylene glycol blend. Typically, it is introduced into the jacketed mixing vessel together with the intermediate polyethylene glycol and the polyethylene glycol 4000 or 6000, the mixture then being stirred until a clear solution is obtained. Other co-solvents, for example propylene glycol, may also be introduced in this way.

The capsule fill is prepared by combining the polyethylene glycol blend, optionally including glycerol and/or one or more other co-solvents, with the benzodiazepine. Typically, the benzodiazepine is simply stirred into the molten polyethylene glycol blend contained in the jacketed mixing vessel, the blend having been brought to a temperature of about 65°C. Stirring is continued until the benzodiazepine has completely dissolved or has become homogeneously suspended, which typically takes from 5 to 15 minutes. Once the benzodiazepine has fully dissolved the mixture may be deaereated, by applying a vacuum to the jacketed mixing vessel. When deaereation is complete, the vacuum is broken, and the mixture for the capsule fill is transferred to a thermostatically controlled jacketed vessel.

When it is desired to introduce chlorophyll into the capsule fill, the chlorophyll is suitably dissolved in warm, purified water to form a solution and this chlorophyll solution is then added to the mixture of benzodiazepine and polyethylene glycols in the jacketed vessel, prior to deaereation. Deaeration is suitably then carried out as described above.

When a conventional two-piece hard gelatin capsule is used, the unit dosage form of the invention is prepared by encapsulating the fill using standard techniques used for filling gels into such shells. Unit dosage forms comprising soft gelatin capsule shells are prepared by delivering the capsule fill from the jacketed vessel, via thermostatically controlled feed tubing, to a specially adapted encapsulation machine where it is encapsulated, typically by a rotary die process, in a soft gelatin shell. The encapsulation machine is suitably an RP Scherer encapsulation machine modified for filling at elevated temperature.

The soft gelatin for the shell is typically prepared batchwise by combining together glycerol, the embrittlement inhibiting composition and purified water, and transferring this blend to a gelatin melter to which gelatin powder is then added. The mixture is generally blended under heat and vacuum for one to two hours until a deaereated homogeneous solution is obtained. The solution is then typically transferred to an electrically heated holding vessel, maintained in the region of 60°C, to which any desired additional ingredients, such as chlorophyll, are then added.

The capsules prepared as described above are typically formulated to contain 10, 15, 20 or 30mg of the benzodiazepine temazepam. The initial mass of the fill of one capsule, prior to encapsulation, is usually from 250 to 300mg, typically about 280mg. However, once encapsulation has taken place the mass of the fill generally increases due to the migration of water and a small amount of glycerol from the shell to the fill, as a consequence of the hydrophilicity of the fill. Thus the total mass of the capsule fill post encapsulation is usually from 280 to 320mg, for example from 290 to 310mg and preferably about 300mg, preferably including 10mg of temazepam. In general the water content of the fill post encapsulation, following water ingress, is in the region of 5 to 6% by weight.

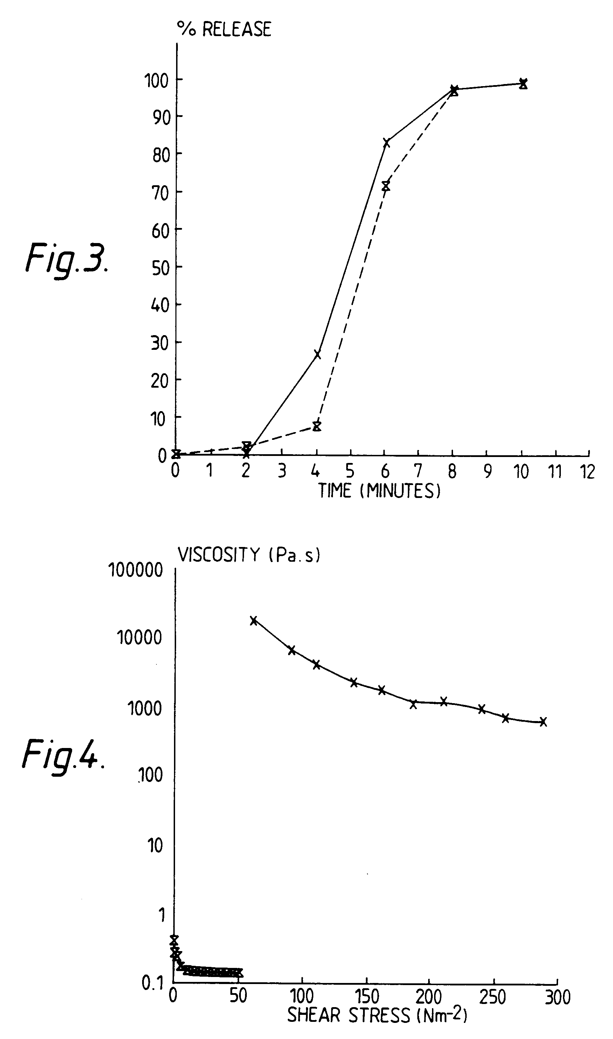

The capsules of the present invention provide an effective deterrent to potential intravenous drug abusers on account of the high viscosity of the capsule fill making injection at ambient temperatures impossible. The dark coloration of both the fill and the capsule shell are further deterrents. There is not, however, any loss of convenience to the genuine user of the benzodiazepine. The dissolution of the drug administered in the capsules of the invention is just as good as that of the original liquid fill temazepam capsules, as illustrated by the in vitro results in Figure 3 of the accompanying drawings. Incorporating a benzodiazepine in a high viscosity polyethylene glycol blend in accordance with the present invention is therefore unlikely to have an adverse effect on the pharmacokinetic properties of the drug in vivo.

Example 6 which follows illustrates how the gel of the invention cannot be expelled from a syringe at ambient temperatures. This contrasts with the thickened compositions based on polyethylene glycol 400 which are shown, in Reference Example 4 which follows, to be capable of being expelled from a syringe at ambient temperature.

The invention is further illustrated by the following Examples. In the accompanying drawings:

Figure 1 shows the variation of viscosity with temperature for a gel of the present invention;

Figure 2 is a histogram illustrating the syringeability of capsule fills based on polyethylene glycol 400;

Figure 3 compares the temazepam in vitro release characteristics of a capsule of the present invention (line X—X) with those of the original liquid fill capsule (line ☒----☒) ; and

Figure 4 is a plot of viscosity against shear stress for the capsule filling of the present invention (line X—X) and for the original liquid fill formulation (line ☒----☒).

Figure 5 is a profile showing the variation of plasma concentration of Temazepam with time for a capsule of the invention (line —g—) and a reference liquid-filled capsule (line ---I---).

Example 1: Preparation of the Polyethylene glycol gel

A batch of the polyethylene glycol gel was prepared as follows. Polyethylene glycol 600 (172.06 kg) was weighed into a jacketed mixing vessel. Polyethylene Glycol 1500 (70.00 kg) and polyethylene glycol 6000 (11.20 kg) were added to the vessel, together with glycerol (14.00 kg), and the mix was heated to about 75°C with stirring. Stirring was then continued (for approximately 1.5 hours) at this temperature until a clear solution was obtained.

The blend of the three polyethylene glycols contained 67.9% by weight of polyethylene glycol 600, 27.6% by weight of polyethylene glycol 1500 and 4.4% by weight of polyethylene glycol 6000.

Example 2: Preparation of capsule fill

To the polyethylene glycol mix prepared according to Example 1, and brought to a temperature of about 65°C, was added temazepam (10.00 kg). Stirring in the jacketed mixing vessel was continued until the temazepam had dissolved.

In order to deaerate the mixture, a vacuum was applied to the mixing vessel. When the required vacuum had been achieved the vacuum was broken. Deaeration was then complete and the mixture was transferred to a thermostatically controlled jacketed vessel.

Example 3: Preparation of coloured capsule fill

The process of Example 2 was repeated, but after complete dissolution of the temazepam a chlorophyll solution was prepared by dissolving chlorophyll KK (0.280 kg) in warm, purified water BP/EP (2.460 kg). This chlorophyll solution was added to the temazepam mixture and stirring was continued for approximately 10 minutes. The mixture was then deaerated as described in Example 2 and transferred to a thermostatically controlled jacketed vessel.

Reference Example 1: Preparation of soft gelatin batch for capsule shell

Glycerol (89.78 kg), Anidrisorb 85/70 (35.255 kg) and purified water BP/EP (157.116 kg) were weighed into a clean, tared and labelled stainless steel mixing vessel fitted with a load cell. Gelatin powder (191.22 kg) was weighed into a second stainless steel vessel fitted with a load cell.

The water/glycerol blend was transferred to a gelatin melter, to which was then added the gelatin powder. The mixture was blended in the gelatin melter under heat and a vacuum of approximately 850 millibars (for 1 to 2 hours) until a deaerated homogeneous solution was obtained. This solution was then transferred to an electrically heated stainless steel holding vessel maintained at approximately 60°C.

Reference Example 2: Preparation of coloured soft gelatin batch

The process of Reference Example 1 was repeated and then to the gelatin solution in the holding vessel were added chlorophyll KK (0.10 kg) dissolved in water and a suspension of titanium dioxide powder (0.25 kg) in glycerol (0.75 kg). These additional ingredients were incorporated into the gelatin solution using a high speed blender, the mixture being maintained at 60°C throughout.

The formulation of the product was as follows:

| Ingredient | kg per 100kg | % wet mass | kg per batch |
|---|---|---|---|
| Gelatin | 40.70 | 40.257 | 191.221 |
| Glycerol | 18.36 | 18.902* | 89.785 |
| Purified Water | 33.44 | 33.077 | 157.116 |
| Anidrisorb 85/70 | 7.50 | 7.418 | 35.235 |
| TiO$_2$ powder | 0.25 | 0.247 | 1.173 |
| Glycerol | 0.75* | *included above | |
| Chlorophyll KK | 0.1 | 0.099 | 0.470 |
| TOTAL | 101.10 | 100.000 | 495.000 |

Example 4: Preparation of Capsules

The fill prepared by the process of Example 2 was delivered via thermostatically controlled feed tubing to an RP Scherer encapsulation machine where it was encapsulated, by the rotary die process using a 5 oval softgel die, in the soft gelatin prepared in Reference Example 2. During the encapsulation the gelatin was lubricated to aid processing.

Initially, i.e. prior to water and/or glycerol ingress, the fill of each capsule weighed 280.000mg. The quantity of each component of the fill per capsule is as follows:

| Ingredient | mg/capsule |
|---|---|
| Temazepam | 10.000 |
| Polyethylene Glycol 600 | 172.060 |
| Polyethylene Glycol 1500 | 70.000 |
| Polyethylene Glycol 6000 | 11.200 |
| Purified water BP/EP | 2.460 |
| Glycerol | 14.000 |
| Chlorophyll KK | 0.280 |
| TOTAL | 280.000 |

The filled soft capsules produced were washed in Solvent 30 and transferred to a tumble drier, where they were tumble dried for approximately one hour in a low humidity air flow at a temperature of about 21°C. Final drying was completed by spreading the soft capsules out on shallow trays and allowing them to dry in a low humidity environment.

It is to be stressed that the above formulation applies to the fill <u>before</u> water and/or glycerol have migrated into it from the shell. The water content will typically increase to form from 4 to 6% of the final mass of the fill, and the glycerol content will increase gradually throughout the shell-life of the capsules.

Example 5: Testing of the Polyethylene glycol gels

Polyethylene glycol compositions of varying formulation were prepared in the laboratory by a process analogous to that of Example 1. In some cases polyethylene glycol 6000 was replaced by polyethylene glycol 4000. The drop point (°C) of each composition was then measured using a standard Metler FP83 drop point apparatus. The results obtained are listed in Table 1 below.

TABLE 1

| Formulation | PEG 600 (wt %) | PEG 1500 (wt %) | PEG 4000 (wt %) | PEG 6000 (wt %) | Drop point (°C) |
|---|---|---|---|---|---|
| A | 68.0 | 28.0 | -- | 4.0 | 48.9 |
| B | 67.9 | 27.6 | -- | 4.4 | 48.2 |
| C | 66.9 | 28.3 | -- | 5.9 | 50.3 |
| D | 73.6 | 21.7 | -- | 4.7 | 48.8 |
| E | 63.3 | 32.6 | -- | 4.1 | 49.0 |
| F | 65.0 | 25.0 | 10.0 | -- | 48.6 |
| G | 63.0 | 30.0 | 7.0 | -- | 48.4 |

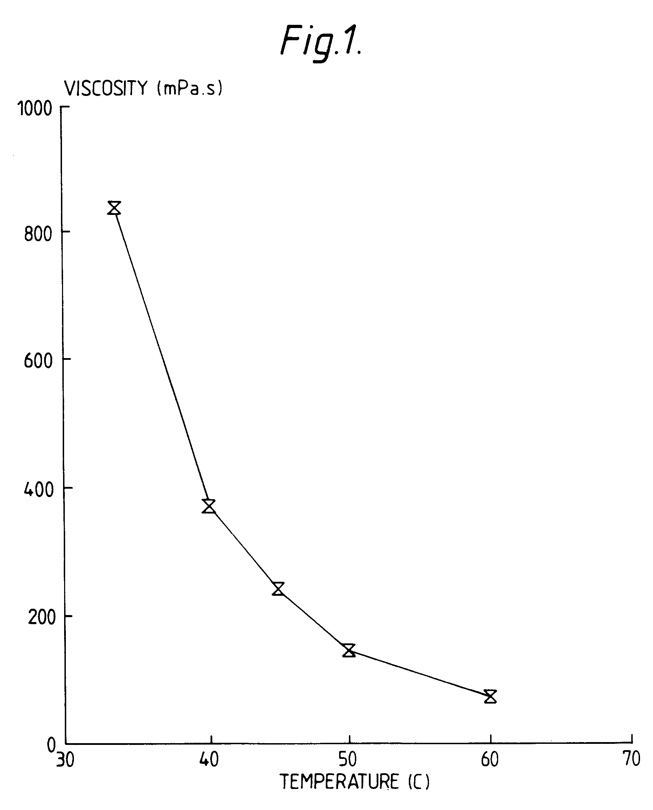

The variation of viscosity with temperature of formulation A was measured using a Contraves Rheomat viscometer, and is illustrated in Figure 1 of the accompanying drawings.

Reference Example 3: Preparation of compositions based on PEG 400

A standard polyethylene glycol 400 temazepam formulation H, corresponding to the original liquid fill formulation and shown below, was prepared by melting polyethylene glycol 400, cooling the temperature to about 45°C, then adding water and glycerol followed by temazepam. The mixture was stirred until all the temazepam had dissolved. The following quantities refer to an amount corresponding to one 10mg temazepam capsule, containing 255mg of fill (prior to water or glycerol ingress).

| Ingredients of H | mg | wt % |
|---|---|---|
| Polyethylene Glycol 400 | 230.00 | 90.196 |
| Glycerol | 12.75 | 5.000 |
| Water | 2.25 | 0.883 |
| Temazepam | 10.00 | 3.921 |
| TOTAL | 255.00 | 100.00 |

Six formulations I to N based on H were then prepared, using the same amount of temazepam, glycerol and water but replacing part of the polyethylene glycol 400 by varying amounts of polyethylene glycols 1500, 4000 and/or 6000, as thickener. The particular polyethylene glycol blends used in each formulation is shown in Table 2, which follows:

TABLE 2

| Formulation | PEG 400 | | PEG 1500 | | PEG 4000 | | PEG 6000 | |
|---|---|---|---|---|---|---|---|---|
| | mg | wt% | mg | wt% | mg | wt% | mg | wt% |
| H | 230.00 | 100.00 | --- | --- | --- | --- | --- | --- |
| I | 209.60 | 91.1 | --- | --- | 20.4 | 8.9 | --- | --- |
| J | 186.65 | 81.2 | 38.25 | 16.6 | 5.10 | 2.2 | --- | --- |
| K | 189.20 | 82.3 | 38.25 | 16.6 | --- | --- | 2.55 | 1.1 |
| L | 213.90 | 93.0 | --- | --- | 10.35 | 4.5 | 5.75 | 2.5 |
| M | 214.59 | 93.3 | --- | --- | --- | --- | 15.41 | 6.7 |
| N | 179.00 | 77.8 | 51.0 | 22.2 | --- | --- | --- | --- |

Reference Example 4: Testing of Compositions based on PEG 400

Formulations H to N prepared in Reference Example 3 were tested for their syringeability as follows.

A JJ tensile strength tester was used to measure the compression force required to push an equivalent volume of each formulation at 20°C through a 2ml syringe and 25G needle. The syringe was filled with the sample and placed in the purpose-built holder. A load cell of 500N was lowered onto the syringe at 10mm/minute. At the instant a load was registered a stop clock was started. After 3 minutes the maximum load was recorded.

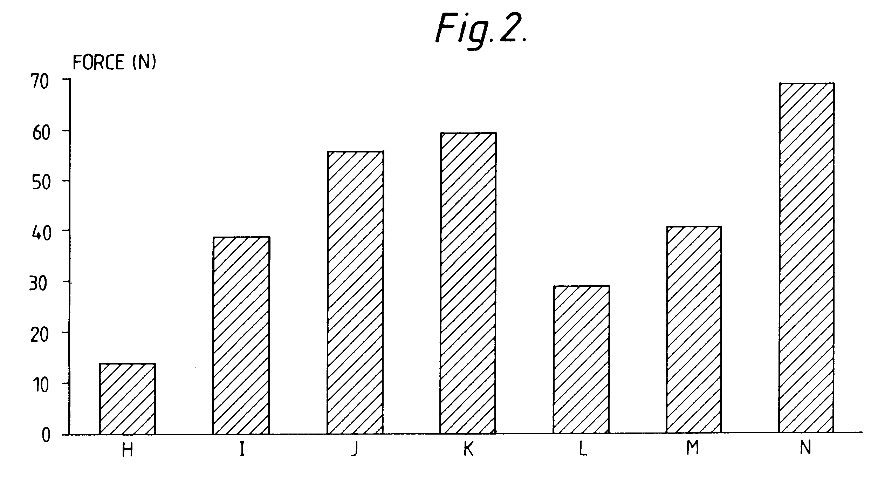

The results are shown in Table 3 below and in the histogram of Figure 2 of the accompanying drawings. All the formulations were capable of being expelled from a syringe at ambient temperature.

TABLE 3

| Formulation | Average Force (n = 3) | | |
|---|---|---|---|
| | Newtons | (S.D., | C.V.) |
| H | 13.7 | (0.6, | 4.2) |
| I | 38.7 | (0.6, | 1.5) |
| J | 55.7 | (1.2, | 2.1) |
| K | 59.3 | (1.2, | 1.9) |
| L | 29.0 | (1.0, | 3.4) |
| M | 40.7 | (2.1, | 5.1) |
| N | 69.3 | (1.2, | 1.7) |

Example 6: Further testing of the Polyethylene glycol gels

Temazepam capsules comprising formulation A of the invention, prepared in Example 5, were compared with temazepam capsules comprising formulation H, prepared in Reference Example 3, with respect to their properties of dissolution (temazepam release). The viscosity of formulations A and H were then compared and finally the syringeability of formulation A was compared with that of formulations H to N based on polyethylene glycol 400, as described in Reference Examples 3 and 4.

Dissolution

The dissolution of 20mg temazepam capsules comprising A, and 20mg temazepam capsules comprising H, was determined using the U.S.P. dissolution test. Dissolution was measured in 0.1M HCl using a standard flow through technique (Apparatus: Apple II Europlus Microcomputer; LKB Ultrospec 4052 TDS Spectrophotometer; Caleva Dissolution Apparatus, model 6ST; and Watson-Marlow 5025 Peristaltic Pump. Parameters: USP Paddle method at 100 r.p.m; dissolution medium, 0.1M HCl; bath temperature, 37°C; run time, 10 minutes; sample time, 1 minutes; and wavelength, 230nm).

The dissolution profiles (average of 6 individual determinations) are shown in the graph of % temazepam release against time in Figure 3 of the accompanying drawings. The profiles indicates that there is no reduction in temazepam release from capsules comprising formulation A as compared with capsules comprising formulation H, containing only polyethylene glycol 400.

Viscosity

The flow properties of formulations A and H, after encapsulation, were compared by measuring the variation of viscosity with shear stress for each using a Carri-Med CSL Rheometer.

The results are shown in the graph in Figure 4 of the accompanying drawings. The graph clearly demonstrates the superiority of formulation A of the invention over formulation H in an application where high viscosity is needed.

Syringeability

At ambient temperature (20°C) it proved impossible to draw formulation A up into a 2ml syringe, or to inject it through a 25G needle. Measurement of the compression force required to pass the formulation through such a needle, by the method described in Reference Example 4, also proved impossible at ambient temperature since the needle was consistently blown off. It proved necessary to increase the temperature of Formulation A to at least 50°C (see Figure 2) for it to begin to exhibit the viscosity and syringeability characteristics of the unthickened polyethylene glycol 400 formulation H.

Example 7: Pharmacokinetic study of Temazepam Capsules

A study was carried out to define and to compare the pharmacokinetic characteristics of Temazepam capsules of the invention with conventional liquid-filled capsules, which are subject to intravenous abuse, following their oral administration to human subjects.

The open, randomized, cross-over study involved administration to 24 healthy volunteers of a unit dosage form of the invention, comprising Temazepam in a soft gelatin capsule, and of a liquid-filled reference formulation of Temazepam. In each case a single oral dose of 20mg of the drug was provided.

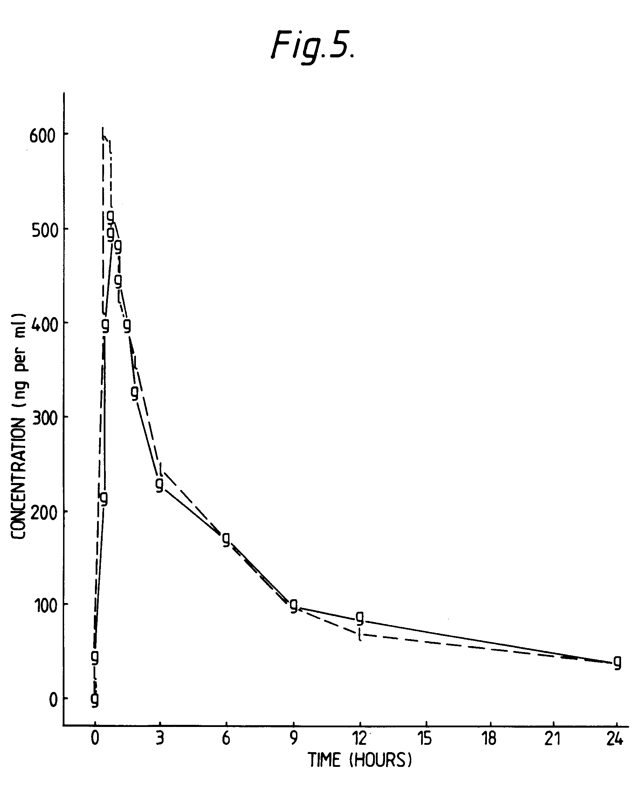

The results of the study are shown in accompanying Figure 5, in which the line (—g—) denotes capsules of the invention (TG) and the line (---l---) denotes the reference formulation (TL).

The relative bioavailability of the two formulations assessed in terms of the area under the time versus plasma concentration profile is not significantly different from parity and lies well within the acceptable 80-120% limits for bioequivalence. Although the mean Cmax for TG (616.6ng/ml) may be 12.9% lower than for TL (707.9ng/ml) and the median time to reach Cmax (Tmax) s 40 min (TG) vs 30 min (TL), there is no significant difference between TG and TL either in their absorption constant (Ka) (0.123 vs 0.138 min-1 respectively) or their distribution (alpha) (29.5 vs 32.4 min) and elimination (beta) (6.3 vs 6.6 h) half-lives.

The results therefore show that the increased viscosity of the capsule fill in the unit dosage form of the invention does not adversely affect the pharmacokinetic characteristics of the encapsulated Temazepam. In particular a rapid rise in plasma concentration followed by a prompt fall is seen to be conserved, which is essential for the use of Temazepam as a hypnotic and premedicant.

## Claims

1.  A pharmaceutical unit dosage form comprising a soft gelatin capsule shell or a two-piece hard gelatin capsule and a fill comprising a benzodiazepine dissolved or suspended in a gel, wherein the gel has a drop point (°C), prior to encapsulation, of at least 46 and comprises at least 63% by weight of polyethylene glycol 600, at least 2% by weight of polyethylene glycol 4000 or 6000 and at least 21% by weight of an intermediate polyethylene glycol having an average molecular weight between 600 and 4000, the gel consisting of polyethylene glycol.

2.  A unit dosage form according to claim 1 wherein the intermediate polyethylene glycol is polyethylene glycol 1500.

3.  A unit dosage form according to claim 1 or 2 wherein the gel comprises 67 to 68% by weight of polyethylene glycol 600, 27 to 28% by weight of polyethylene glycol 1500 and 4 to 6% by weight of polyethylene glycol 6000.

4.  A unit dosage form according to any one of the preceding claims, wherein the gel has a drop point prior to encapsulation of 48°C to 60°C.

5.  A unit dosage form according to claim 4, wherein the drop point is from 48.2°C to 50.3°C.

6.  A unit dosage form according to any one of the preceding claims wherein the fill further comprises glycerol or any other co-solvent.

7.  A unit dosage form according to claim 6 wherein the fill comprises from 2 to 20% by weight of glycerol.

8.  A unit dosage form according to any one of the preceding claims wherein the benzodiazepine is temazepam.

9.  A unit dosage form according to any one of the preceding claims wherein the fill further comprises a pigment or dye.

10. A unit dosage form according to claim 9 wherein the pigment is chlorophyll.

11. A process for the preparation of a pharmaceutical unit dosage form as claimed in any one of claims 1 to 10, which process comprises:
    a) preparing a polyethylene glycol blend by combining at least 63% by weight of polyethylene glycol 600, at least 2% by weight of polyethylene glycol 4000 or 6000 and at least 21% by weight of an intermediate polyethylene glycol;
    b) forming a fill by dissolving or suspending a benzodiazepine in the polyethylene glycol blend, and
    c) encapsulating the fill in a soft gelatin capsule shell or a two-piece gelatin capsule.

## Patentansprüche

1.  Form einer pharmazeutischen Dosiseinheit, die eine weiche Gelatinekapselschale oder eine zweistückige harte Gelatinekapsel und eine Füllung umfaßt, welche ein Benzodiazepin in einem Gel gelöst oder suspendiert enthält, wobei das Gel vor der Einkapselung einen Tropfpunkt (°C) von mindestens 46 hat und mindestens 63 Gew.% Polyethylenglykol 600, mindestens 2 Gew.% Polyethylenglykol 4000 oder 6000 und mindestens 21 Gew.% intermediärer Polyethylenglykol, das ein durchschnittliches Molekulargewicht zwischen 600 und 4000 hat, enthält, das Gel also aus Polyethylenglykol besteht.

2.  Form einer Dosiseinheit nach Anspruch 1, in der das intermediäre Polyethylenglykol Polyethylenglykol 1500 ist.

3.  Form einer Dosiseinheit nach Anspruch 1 oder 2, in der das Gel 67 bis 68 Gew.% Polyethylenglykol 600; 27 bis 28 Gew.% Polyethylenglykol 1500 und 4 bis 6 Gew.% Polyethylenglykol 6000 enthält.

4. Form einer Dosiseinheit nach einem der vorangehenden Ansprüche, in der das Gel vor der Einkapselung einen Tropfpunkt von 48°C bis 60°C hat.

5. Form einer Dosiseinheit nach Anspruch 4, in der der Tropfpunkt zwischen 48,2°C und 50,3°C liegt.

6. Form einer Dosiseinheit nach einem der vorangehenden Ansprüche, in der die Füllung außerdem Glycerin oder ein anderes Co-Lösungsmittel enthält.

7. Form einer Dosiseinheit nach Anspruch 6, in der die Füllung 2 bis 20 Gew.% Glycerin enthält.

8. Form einer Dosiseinheit nach einem der vorangehenden Ansprüche, in der das Benzodiazepin Temazepam ist.

9. Form einer Dosiseinheit nach einem der vorangehenden Ansprüche, in der die Füllung außerdem ein Pigment oder einen Farbstoff enthält.

10. Form einer Dosiseinheit nach Anspruch 9, in der das Pigment Chlorophyll ist.

11. Verfahren zur Herstellung einer Form einer pharmazeutischen Dosiseinheit, wie sie in einem der vorangehenden Ansprüche beansprucht ist, das die folgenden Schritte umfaßt:
a) Herstellen einer Polyethylenglykol-Mischung durch Kombination von mindestens 63 Gew.% Polyethylenglykol 600, mindestens 2 Gew.% Polyethylenglykol 4000 oder 6000 und mindestens 21 Gew.% intermediäres Polyethylenglykol;
b) Bilden einer Füllung durch Lösen oder Suspendieren eines Benzodiazepin in der Polyethylenglykol-Mischung, und
c) Einkapseln der Füllung in einer weichen Gelatinekapselschale oder einer zweistückigen Gelatinekapsel.

**Revendications**

1. Forme de dosage unitaire pharmaceutique, comprenant une enveloppe de gélule en gélatine molle, ou une gélule en gélatine dure en deux pièces et une charge formée d'une benzodiazépine dissoute ou en suspension dans un gel, où le gel possède un point de goutte (°C), avant l'encapsulation, d'au moins 46 et comprend au moins 63% en poids de polyéthylèneglycol 600, au moins 2% en poids de polyéthylèneglycol 4000 ou 6000 et au moins 21% en poids d'un polyéthylèneglycol intermédiaire possédant un poids moléculaire moyen compris entre 600 et 4000, le gel étant constitué de polyéthylèneglycol.

2. Forme de dosage unitaire suivant la revendication 1, caractérisée en ce que le polyéthylèneglycol intermédiaire est le polyéthylèneglycol 1500.

3. Forme de dosage unitaire suivant la revendication 1 ou 2, caractérisée en ce que le gel comprend 67 à 68% en poids de polyéthylèneglycol 600, 27 à 28% en poids de polyéthylèneglycol 1500 et 4 à 6% en poids de polyéthylèneglycol 6000.

4. Forme de dosage unitaire suivant l'une quelconque des revendications précédentes, caractérisée en ce que le gel possède un point de goutte, avant l'encapsulation, de 48°C à 60°C.

5. Forme de dosage unitaire suivant la revendication 4, caractérisée en ce que le point de goutte varie de 48,2°C à 50,3°C.

6. Forme de dosage unitaire suivant l'une quelconque des revendications précédentes, caractérisée en ce que la charge comprend en outre du glycérol ou tout autre cosolvant.

7. Forme de dosage suivant la revendication 6, caractérisée en ce que la charge comprend 2 à 20% en poids de glycérol.

**8.** Forme de dosage unitaire suivant l'une quelconque des revendications précédentes, caractérisée en ce que la benzodiazépine est le témazépam.

**9.** Forme de dosage unitaire suivant l'une quelconque des revendications précédentes, caractérisée en ce que la charge comprend aussi un pigment ou un colorant.

**10.** Forme de dosage unitaire suivant la revendication 9, caractérisée en ce que le pigment est la chlorophylle.

**11.** Procédé de préparation d'une forme de dosage unitaire pharmaceutique suivant l'une quelconque des revendications 1 à 10, caractérisé en ce qu'il comprend:

a) la préparation d'un mélange de polyéthylèneglycols en combinant au moins 63% en poids de polyéthylèneglycol 600, au moins 2% en poids de polyéthylèneglycol 4000 ou 6000 et au moins 21% en poids d'un polyéthylèneglycol intermédiaire,

b) la formation d'une charge en dissolvant ou en mettant en suspension une benzodiazépine dans le mélange de polyéthylèneglycols et

c) l'encapsulation de la charge dans une enveloppe de gélule en gélatine molle, ou dans une gélule en gélatine en deux pièces.

# Fig.1.

Fig.2.

EP 0 502 042 B1

Fig.3.

% RELEASE

TIME (MINUTES)

Fig.4.

VISCOSITY (Pa.s)

SHEAR STRESS (Nm-2)

# Fig.5.